# EUROPÄISCHE PATENTSCHRIFT

(11) **EP 2 987 571 B1**
(45) Veröffentlichungstag und Bekanntmachung des Hinweises auf die Patenterteilung: **15.08.2018**
(21) Anmeldenummer: 14181352.7
(22) Anmeldetag: 19.08.2014
(51) Int. Cl.: B22F 1/00, C22F 1/14, B22F 9/24, C07C 53/10

(54) **Verfahren zur Herstellung von aktivem Palladium(0)-Pulver, aktives Palladium(0)-Pulver und seiner Verwendung für die Herstellung eines Palladiumsalzes**
Method for the production of active palladium (0) powder, active palladium (0) powder and its use in making a palladium salt
Procédé de fabrication de poudre de palladium(0) active, poudre de palladium(0) active et son utilisation pour la fabrication d'un sel de palladium

(43) Veröffentlichungstag der Anmeldung: 24.02.2016
(73) Patentinhaber: Heraeus Deutschland GmbH & Co. KG, 63450 Hanau (DE)
(72) Erfinder: Stettner, Martin, 63674 Altenstadt (DE); Von Eiff, Hermann, 63453 Neuberg (DE); Thiel, Vasco, 63755 Alzenau (DE); Voß, Steffen, 63694 Limeshain (DE)
(74) Vertreter: Maiwald Patent- und Rechtsanwaltsgesellschaft mbH

(56) Entgegenhaltungen:
- WO-A1-2012/169992
- W. E. MODDEMAN ET AL: "XPS surface and bulk studies of heat treated palladium in the presence of hydrogen at 150°C", SURFACE AND INTERFACE ANALYSIS, Bd. 11, Nr. 6-7, 1. April 1988 (1988-04-01), Seiten 317-326, XP055172858, ISSN: 0142-2421, DOI: 10.1002/sia.740110609
- P D Cobden ET AL: "Formation and Decomposition of Palladium Hvdride Particles - IMAGING PICTURES ON THE NANOMETRE SCALE", Platinum Metals Review, 1. Oktober 1998 (1998-10-01), Seiten 141-144, XP055173945, Gefunden im Internet: URL:http://www.technology.matthey.com/pdf/ pmr-v42-i4-141-144.pdf [gefunden am 2015-03-04]

## Beschreibung

Pulverförmiges Palladium findet viele unterschiedliche Anwendungen, beispielsweise als Katalysator oder auch als Edukt für die Umsetzung mit geeigneten Reaktionspartnern.

Palladium(0)-Pulver (d.h. pulverförmiges, metallisches Palladium der Oxidationsstufe 0) bzw. Palladium(0)-Schwämme finden unter anderem Anwendung für die Synthese von Palladiumsalzen wie z.B. Palladium(II)-nitrat oder Palladium(II)-carboxylate (z.B. Palladium(II)-acetat oder Palladium(II)-propionat). Bei der Herstellung von Palladium(II)-nitrat wird Palladium(0)-Pulver beispielsweise mit Salpetersäure umgesetzt. Bei der Herstellung von Palladium(II)-acetat wird Palladium(0)-Pulver beispielsweise mit Essigsäure und Salpetersäure gemäß der folgenden Reaktionsgleichung umgesetzt:

3 Pd + 6 HNO₃ + 6 HOAc → Pd₃(OAc)₆ + 6 NO₂ + 6 H₂O

Für eine effiziente Prozessführung ist erwünscht, dass die Reaktion bereits bei einer relativ niedrigen Temperatur (z.B. bereits bei Raumtemperatur) anspringt und daher ein zusätzliches externes Erhitzen des Ausgangsgemisches nicht erforderlich ist oder zumindest minimiert werden kann. Hierfür ist jedoch erforderlich, dass das Palladium(0)-Pulver für diese Reaktion eine ausreichend hohe Aktivität besitzt.

Es ist allgemein bekannt, dass Palladium(0)-Pulver über verschiedene Herstellungsverfahren zugänglich ist.

Beispielsweise kann Palladium(0)-Pulver durch thermische Zersetzung von Diamindichloropalladium(II) hergestellt werden. Auch über die Reduktion von Hexa- oder Tetrachloropalladat mit Ameisensäure ist Palladium(0)-Pulver erhältlich.

Weiterhin ist allgemein bekannt, halogenhaltige Palladiumverbindungen mittels Hydrazin zu Palladium zu reduzieren. So wird beispielsweise in der DE 102 49 521 ein Verfahren zur Herstellung von Palladium beschrieben, in dem eine halogenhaltige Palladiumverbindung mittels Hydrazin und/oder deren Derivaten zu Palladium(0)-Pulver reduziert wird und das erhaltene Palladium(0)-Pulver unter Stickstoffatmosphäre auf eine Temperatur von 550-1200°C erhitzt wird.

W.E. Moddeman et al., Surface and Interface Analysis, Vol. 11, 1988, S. 317-326, beschreiben ein Verfahren zur Herstellung eines Pd(0)-Pulvers, in dem ein Pd(0)-Ausgangspulver in einer Wasserstoffgasatmosphäre bei 150°C erhitzt wird. Das über die H₂-Behandlung bei 150°C erhaltene Pd(0)-Pulver wird für die Gewinnung von möglichst reinem Wasserstoff verwendet.

Es hat sich jedoch gezeigt, dass die nach den herkömmlichen Verfahren hergestellten Palladium(0)-Pulver nicht ausreichend aktiv sind, um bei der Herstellung von Palladiumsalzen wie z.B. Palladium(II)-nitrat oder Palladium(II)-carboxylaten die Reaktion bereits bei einer relativ niedrigen Temperatur (bevorzugt bereits bei Raumtemperatur) zu starten.

Eine Aufgabe der vorliegenden Erfindung besteht daher in der Bereitstellung eines Palladium(0)-Pulvers, das eine möglichst hohe Aktivität aufweist, insbesondere für die Herstellung von Palladiumsalzen.

Gelöst wird diese Aufgabe durch ein Verfahren zur Herstellung von Palladium(0)-Pulver, wobei ein Palladium(0)-Ausgangspulver in einer Wasserstoffgasatmosphäre in einem Ofen einer thermischen Behandlung bei einer Temperatur im Bereich von 230°C bis 370°C unterzogen wird.

Wie nachfolgend noch eingehender beschrieben wird, wurde im Rahmen der vorliegenden Erfindung überraschend festgestellt, dass bei einer Behandlung eines Pd-Ausgangspulvers in einer Wasserstoffgasatmosphäre bereits relativ niedrige Temperaturen (230°C - 370°C) ausreichend sind, um ein sehr aktives Palladium(0)-Pulver zu erhalten. Mit diesem Palladium(0)-Pulver kann die Umsetzungsreaktion zu einem Palladiumsalz wie z.B. Palladium(II)-nitrat oder Palladium(II)-carboxylat bereits bei Raumtemperatur gestartet werden. Weiterhin wurde im Rahmen der vorliegenden Erfindung überraschend festgestellt, dass ein Einhalten dieser relativ niedrigen Behandlungstemperatur hinsichtlich der Aktivität wesentlich ist und eine thermische Wasserstoffbehandlung bei einer Temperatur oberhalb von 370°C zu einem Palladium(0)-Pulver mit deutlich geringerer Aktivität führt.

Die Temperatur der thermischen Behandlung bezieht sich auf die Temperatur im Offeninnenraum.

Der Begriff "Palladium(0)" bezieht sich auf Palladium der Oxidationszahl 0, d.h. metallisches Palladium.

Im Rahmen der vorliegenden Erfindung umfasst der Begriff "Palladium(0)-Pulver" auch einen Palladium(0)-Schwamm. Wie dem Fachmann bekannt ist, handelt es sich bei einem Palladium-Schwamm um eine relativ grobkörnige Form des Palladiums. Im Rahmen der vorliegenden Erfindung wird unter einem Pulver auch ein solches Material verstanden, bei dem die Pulverpartikel zumindest teilweise zusammengesintert sind und das Material daher zwar partikelförmig, jedoch nicht mehr oder nur teilweise schütt- oder rieselfähig ist.

Verfahren zur Herstellung eines Palladium(0)-Ausgangspulvers sind dem Fachmann bekannt.

Beispielsweise kann das Palladium(0)-Ausgangspulver durch Reduktion einer Pd(II)-Verbindung oder einer Pd(IV)-Verbindung erhalten wird. Bevorzugt handelt es sich um eine halogenhaltige Pd(II)- oder Pd(IV)-Verbindung, wie z.B. PdCl₂, (NH₄)₂PdCl₆, (NH₄)₂PdCl₄, Pd(NH₃)₄Cl₂, Pd(NH₃)₂Cl₂.

Als beispielhafte Reduktionsmittel können Hydrazin, Hydraziniumsalze, organische Hydrazinderivate oder Ameisensäure genannt werden. Die Herstellung von Palladium unter Verwendung von Hydrazin als Reduktionsmittel wird beispielsweise in der DE 102 49 521 A1 beschrieben.

Das Palladium(0)-Ausgangspulver kann auch durch thermische Zersetzung von Diamindichloropalladium(II) hergestellt werden.

Bevor das Palladium(0)-Ausgangspulver der thermischen Wasserstoffbehandlung im Ofen unterzogen wird, kann es optional noch getrocknet werden. Dieser Trocknungsschritt kann beispielsweise in dem Ofen oder auch außerhalb des Ofens erfolgen. Prinzipiell ist es aber auch möglich, dass ein noch feuchtes Palladium(0)-Ausgangspulver der thermischen Wasserstoffbehandlung unterzogen wird.

Wie oben erwähnt, erfolgt die thermische Behandlung des Palladium(0)-Ausgangspulvers in einem Ofen unter einer Wasserstoffgasatmosphäre.

Zur Durchführung der thermischen Behandlung wird das Palladium(0)-Ausgangspulver bevorzugt in einen Ofen eingebracht und man lässt Wasserstoffgas in den Ofen einströmen, so dass das Palladium(0)-Ausgangspulver in einer Wasserstoffgasatmosphäre vorliegt.

Im Rahmen der vorliegenden Erfindung wird unter einem Ofen eine Vorrichtung verstanden, die einen von einer Wand bzw. von Wänden umschlossenen Raum (Ofeninnenraum) aufweist, in dem einem thermisch zu behandelnden Gegenstand in kontrollierter Weise Wärme zugeführt werden kann.

Geeignete Öfen für eine solche thermische Behandlung sind dem Fachmann grundsätzlich bekannt und kommerziell erhältlich. Durch entsprechende Regelungstechnik lässt sich das Aufheizen des Ofens steuern und kontrollieren. Bevorzugt sind die Messelemente zur Temperaturbestimmung so angebracht, dass die Temperatur im Ofeninnenraum verlässlich bestimmt und somit die Gefahr eines Überheizens verringert werden kann. Als beispielhafter Ofen kann ein Rohrofen erwähnt werden. Andere Ofentypen sind aber ebenso geeignet.

Prinzipiell kann der Wasserstoffgehalt der Wasserstoffgasatmosphäre über einen breiten Bereich variieren. Bei niedrigerem Wasserstoffgehalt ist gegebenenfalls eine längere Behandlungsdauer des Palladium(0)-Ausgangspulvers in der Wasserstoffgasatmosphäre zu wählen, um eine ausreichende Aktivität des Palladium(0)-Pulvers (z.B. für eine spätere Umsetzung mit einer Mineralsäure) zu erzielen. Beispielsweise beträgt der Wasserstoffgehalt der Wasserstoffgasatmosphäre (d.h. der Wasserstoffgehalt im Ofeninnenraum) mindestens 5 Vol%, bevorzugter mindestens 10 Vol% oder mindestens 20 Vol%, noch bevorzugter mindestens 30 Vol% oder mindestens 50 Vol%, noch bevorzugter mindestens 70 Vol% oder sogar mindestens 90 Vol%, bezogen auf die Gesamtmenge der in der Wasserstoffgasatmosphäre vorliegenden Gase. Sofern andere Gase vorliegen, kann es sich beispielsweise um inerte Hilfsgase (z.B. N₂) oder unvermeidliche Restmengen an Luft handeln. Der Gehalt an Sauerstoff (z.B. von noch vorhandenen Mengen an Luft) wird bevorzugt so niedrig wie möglich gehalten, z.B. weniger als 1 Vol%, bevorzugter weniger als 0,1 Vol% oder sogar weniger als 0,01 Vol%, bezogen auf die Gesamtmenge der in der Wasserstoffgasatmosphäre vorliegenden Gase

Das Einströmen des Wasserstoffs in den Ofen kann kontinuierlich oder alternativ auch diskontinuierlich erfolgen. Bevorzugt liegt während der gesamten thermischen Behandlung des Palladium(0)-Ausgangspulvers ein kontinuierlicher Wasserstoffdurchfluss im Ofen vor. Wie allerdings später noch erläutert wird, kann es bevorzugt sein, dass nach Beendigung der thermischen Behandlung in der Abkühlphase des Ofens die Wasserstoffzufuhr in den Ofen gestoppt und stattdessen ein inertes Gas wie z.B. Stickstoff während des Abkühlens in den Ofen eingeleitet wird.

Nach der Ausbildung einer Wasserstoffgasatmosphäre im Ofen wird die Ofentemperatur erhöht, wobei aber eine Temperatur von 370°C nicht überschritten werden darf.

Wie bereits oben erwähnt, wird die Aktivität des Palladium(0)-Pulvers im Herstellungsverfahren von Palladiumsalzen deutlich reduziert, wenn die Wasserstoffbehandlung des Palladium(0)-Ausgangspulvers oberhalb von 370°C durchgeführt wird.

Geeignete Maßnahmen, die ein Überheizen eines Ofens verhindern, sind dem Fachmann grundsätzlich bekannt. Beispielsweise kann das Aufheizen des Ofens durch das Fahren einer oder mehrerer Temperaturrampen unterbrochen werden. Bei dem Fahren einer Temperaturrampe wird der Ofen auf eine Haltetemperatur T₁ aufgeheizt und diese Haltetemperatur T₁ wird anschließend für eine Zeitspanne t₁ möglichst konstant gehalten. Wird noch eine zweite Temperaturrampe gefahren, so erfolgt danach ein weiteres Aufheizen von der ersten Haltetemperatur T₁ auf eine zweite Haltetemperatur T₂ und auch diese Haltetemperatur T₂ wird anschließend für eine Zeitspanne t₂ möglichst konstant gehalten. Über das Fahren solcher Temperaturrampen ist eine Annäherung an die vorgegebene Maximaltemperatur des Ofens so möglich, dass die Gefahr eines Überheizens minimiert wird. Die Anzahl der Temperaturrampen, geeignete Haltetemperaturen T₁, T₂, etc. und die Wahl geeigneter Haltezeitspannen t₁, t₂, etc. können vom Fachmann ohne weiteres so ausgewählt werden, dass ein Überheizen des Ofens auf Temperaturen oberhalb von 380°C vermieden wird. Beispielsweise können beim Aufheizen des Ofens auf die Maximaltemperatur 3-10 oder 4-8 Temperaturrampen gefahren werden, wobei die Haltetemperaturen T1, T2, etc. 10-100°C auseinanderliegen können und die Haltezeitspannen t1, t2, etc. 5-90 Minuten oder 15-80 Minuten betragen können. Alternativ oder zusätzlich zu der Anwendung von Temperaturrampen kann die Gefahr eines Überheizens auch durch niedrige Aufheizraten minimiert werden.

Bevorzugt erfolgt die thermische Wasserstoffgasbehandlung in dem Ofen bei einer Temperatur von nicht mehr als 360°C, bevorzugter nicht mehr als 350°C.

Die untere Temperaturgrenze für die thermische Wasserstoffgasbehandlung im Ofen beträgt 230°C, bevorzugter 280°C.

Bevorzugt erfolgt die thermische Behandlung des Palladium(0)-Ausgangspulvers daher bei einer Temperatur im Bereich von 230°C bis 360°C oder von 280°C bis 350°C.

Die Dauer der thermischen Behandlung des Palladium(0)-Ausgangspulvers in der Wasserstoffgasatmosphäre kann über einen breiten Bereich variieren und hängt auch von der Menge des eingesetzten Palladium(0)-Ausgangspulvers ab. Die Dauer der thermischen Behandlung sollte so ausgewählt sein, dass das erhaltene Palladium(0)-Pulver eine ausreichend hohe Aktivität in einem Herstellungsverfahren für Palladiumsalze aufweist. Da die Aktivität eines Palladium(0)-Pulvers für die Herstellung eines Palladiumsalzes ohne weiteres überprüfbar ist (beispielsweise über eine Testreaktion in kleinem Maßstab oder auch über eine thermogravimetrische Analyse des Pd(0)-Pulvers), lässt sich die optimale Zeitdauer für die thermische Behandlung des Pd(0)-Ausgangspulvers problemlos ermitteln.

Nach der thermischen Behandlung lässt man den Ofen abkühlen (z.B. auf Raumtemperatur) und das Palladium(0)-Pulver kann anschließend entnommen und für die Herstellung von Palladiumsalzen eingesetzt werden. Bevorzugt wird während des Abkühlens kein Wasserstoff mehr, sondern ein inertes Gas wie z.B. Stickstoff oder ein Edelgas in den Ofen eingeleitet.

Nach dem Abkühlen des Ofens kann das Palladium(0)-Pulver noch einer Nachbehandlung unterzogen werden, beispielsweise einer mechanischen Zerkleinerung bzw. einem Mahlverfahren.

Sofern das Palladium(0)-Pulver nicht unmittelbar für die Herstellung eines Palladiumsalzes verwendet wird, kann es vorteilhaft sein, das Palladium(0)-Pulver in einer inerten Gasatmosphäre (z.B. einer N₂-Atmosphäre) zu lagern.

Wie nachfolgend noch eingehender beschrieben wird, zeigt das in dem erfindungsgemäßen Verfahren hergestellte Palladium(0)-Pulver nicht nur eine verbesserte Aktivität bei der Herstellung von Palladiumsalzen, sondern weist auch eine charakteristische Massenzunahme beim Aufheizen bzw. Glühen an Luft auf, die es von anderen Palladium(0)-Pulvern unterscheidet.

Das Palladium(0)-Pulver der vorliegenden Erfindung weist daher beim Aufheizen an Luft bis zu einer Temperatur von 990°C eine Massenzunahme von mindestens 13,0 Gew% auf.

Die Massenzunahme kann über Thermogravimetrie bestimmt werden. Die Aufheizrate beträgt z.B. 10°C/min. Das Palladium(0)-Pulver wird von einer Starttemperatur, die üblicherweise bei 25°C liegt, bis zu einer Temperatur von 990°C aufgeheizt und die in diesem Temperaturintervall erfolgende Massenzunahme wird bestimmt. Die thermogravimetrische Messung erfolgt in einer Luftatmosphäre.

Bevorzugt beträgt die Massenzunahme mindestens 13,5 Gew%, bevorzugter mindestens 14,0 Gew%.

Anhand dieser charakteristischen Massenzunahme des erfindungsgemäßen Palladium(0)-Pulvers ist es auch möglich, über eine thermogravimetrische Analyse sehr schnell zu bestimmen, ob ein bestimmtes Palladium(0)-Pulver eine ausreichend hohe Aktivität für die Herstellung von Palladiumsalzen zeigt.

In einem weiteren Aspekt betrifft die vorliegende Erfindung die Verwendung des oben beschriebenen Palladium(0)-Pulvers als Edukt für die Herstellung eines Palladiumsalzes.

Bei dem Palladiumsalz kann es sich um ein Palladium(II)-salz oder auch um ein Palladium(IV)-salz handeln.

Beispielhafte Salze sind Palladiumsalze von Mineralsäuren (z.B. Pd(II)-nitrat, Pd(II)-sulfat oder Pd(II)-chlorid) und Palladium(II)-salze von Carbonsäuren (z.B. C₂₋₈-Carbonsäuren) wie z.B. Palladiumacetat oder Palladiumpropionat.

In einem weiteren Aspekt betrifft die vorliegende Erfindung ein Verfahren zur Herstellung eines Palladiumsalzes, umfassend
(i) die Bereitstellung eines Palladium(0)-Pulvers gemäß dem oben beschriebenen Verfahren, und
(ii) die Umsetzung des Palladium(0)-Pulvers mit einer Mineralsäure.

Wie bereits oben erwähnt, kann es sich bei dem Palladiumsalz um ein Palladium(II)-salz oder auch um ein Palladium(IV)-salz handeln. Hinsichtlich beispielhafter Salze kann auf die obigen Ausführungen verwiesen werden.

In Schritt (i) erfolgt die Bereitstellung eines Palladium(0)-Pulvers gemäß dem oben beschriebenen Verfahren.

In Schritt (ii) wird das Palladium(0)-Pulver mit einer Mineralsäure umgesetzt. Geeignete Reaktionsbedingungen für die Umsetzung des Palladium(0)-Pulvers mit einer Mineralsäure sind dem Fachmann bekannt. Geeignete Mineralsäuren sind beispielsweise Salpetersäure, Schwefelsäure, Salzsäure oder ein Gemisch von mindestens zwei dieser Mineralsäuren (z.B. ein Gemisch aus Salpetersäure und Salzsäure). Eine bevorzugte Mineralsäure ist Salpetersäure.

In einer bevorzugten Ausführungsform handelt es sich um ein Verfahren zur Herstellung von Palladium(II)-nitrat, wobei das Palladium(0)-Pulver in Schritt (ii) mit Salpetersäure umgesetzt wird. Das Palladium(II)-nitrat kann für weitere Umsetzungen verwendet werden, beispielsweise für die Herstellung weiterer Salze, indem das Nitrat gegen ein anderes Anion ausgetauscht wird.

Im Rahmen der vorliegenden Erfindung ist es auch möglich, dass in Schritt (ii) zusätzlich zu der Mineralsäure (z.B. Salpetersäure) noch mindestens ein weiterer Reaktionspartner anwesend ist. In einer bevorzugten Ausführungsform erfolgt die Umsetzung in Schritt (ii) in Anwesenheit einer Carbonsäure oder eines Carbonsäureanhydrids oder eines Gemisches davon. Dadurch lässt sich ein Palladium(II)-carboxylat herstellen.

Bevorzugt handelt es sich bei dem Palladium(II)-carboxylat um ein Palladium(II)-C₂₋₈carboxylat wie z.B. Palladiumacetat oder Palladiumpropionat. Entsprechend handelt es sich bei der Carbonsäure bevorzugt um eine C₂₋₈-Carbonsäure wie z.B. Essigsäure oder Propionsäure. Es können auch Anhydride dieser Carbonsäuren in Schritt (ii) anwesend sein.

In einer bevorzugten Ausführungsform handelt es sich bei der Mineralsäure um Salpetersäure und bei der Carbonsäure um Essigsäure. Durch die Anwesenheit dieser Reaktionspartner in Schritt (ii) wird Palladium(II)-acetat erhalten.

Geeignete Reaktionsbedingungen für die Umsetzung des Palladium(0)-Pulvers mit einer Mineralsäure (wie z.B. Salpetersäure) und einer Carbonsäure (wie z.B. Essigsäure) sind dem Fachmann bekannt.

Bei Verwendung des erfindungsgemäßen Palladium(0)-Pulvers springt die Reaktion bereits bei einer relativ niedrigen Temperatur, z.B. bereits bei Raumtemperatur, an.

Gegebenenfalls kann das die Edukte enthaltende Ausgangsgemisch etwas erhitzt werden, um die Reaktion zu starten.

Die Erfindung wird anhand der folgenden Beispiele eingehender erläutert.

### Beispiele

Für alle folgenden Versuche wurde dasselbe Palladium(0)-Ausgangspulver verwendet, das gemäß dem Beispiel der DE 102 49 521 A1 folgendermaßen hergestellt wurde:
Pd(NH₃)2Cl₂ wird in ein Becherglas überführt und mit so viel heißem, deionisierten Wasser versetzt, bis sich die Suspension gut rühren lässt. Anschließend gibt man unter weiterem Rühren 5-10 ml Ammoniaklösung (25%ig) hinzu, so dass eine leicht alkalische Lösung entsteht. Anschließend wird langsam und portionsweise 30-60 ml Hydrazinlösung (22%ig) zugesetzt. Während der Hydrazinzugabe schäumt die Suspension. Die Hydrazinzugabe ist an die Schaumentwicklung anzupassen. Es werden nochmals 3 ml Hydrazinlösung als Überschuss zugegeben. Anschliessend lässt man noch eine Stunde nachrühren und filtriert den entstandenen Pd-Schwamm auf einer Nutsche ab. Der Pd-Schwamm wird mit heißem, deionisierten Wasser ca. 10-mal gewaschen. Der noch leicht feuchte Pd-Schwamm wird in Quarzglasschiffchen überführt und dieses in einen verschließbaren Rohrofen geschoben. Der Ofen ist mit einem Quarzglasinnenrohr ausgestattet. Anschliessend wird Stickstoffgas durch das Innenrohr geleitet. Der Ausgang des Rohres ist mit einer Gaswaschflasche verbunden, die mit 2n Schwefelsäure gefüllt ist. Nach einer Zeit von 10 Minuten, in der der Sauerstoff vollständig aus dem Innenrohr verdrängt wird, wird der Ofen linear innerhalb von zwei Stunden auf eine Temperatur von 250°C aufgeheizt. Diese Temperatur wird für 4 Stunden gehalten, anschließend wird weiter linear auf eine Temperatur von 600-650°C aufgeheizt. Nach einer Haltezeit von 5 Stunden lässt man den Ofen unter Stickstoffspülung auf ca. 50°C abkühlen. Der Pd-Schwamm wird entnommen und mechanisch zerkleinert.

### Vergleichsbeispiel 1

Das nach dem oben beschriebenen Verfahren hergestellte Palladium(0)-Ausgangspulver wurde hinsichtlich seiner Aktivität bei der Herstellung von Palladiumacetat getestet. Hierzu wurde folgendermaßen vorgegangen:
30 g des Palladium(0)-Ausgangspulvers wurden mit 30 ml Essigsäureanhydrid und 300 ml Essigsäure versetzt. Danach erfolgte die Zugabe von Salpetersäure.

Bei Raumtemperatur zeigte sich keine NOₓ-Entwicklung und das Palladium(0)-Pulver reagiert nicht mit Essigsäure und Salpetersäure zu Palladiumacetat. Auch durch ein Erhitzen auf 60°C konnte die Reaktion nicht gestartet werden.

### Beispiel 1

Das Palladium(0)-Ausgangspulver wurde in einen Rohrofen gegeben. Man ließ Wasserstoff in den Ofen einströmen. Der H₂-Durchfluss betrug 2 m³/h. Nach der Ausbildung der Wasserstoffgasatmosphäre wurde der Ofen mit dem folgenden Temperaturprogramm auf eine Maximaltemperatur von 340°C aufgeheizt:
- Aufheizen bis 100°C,
- Halten der Temperatur von 100°C für 60 Minuten (erste Temperaturrampe),
- weiteres Aufheizen bis 150°C,
- Halten der Temperatur von 150°C für 30 Minuten (zweite Temperaturrampe),
- weiteres Aufheizen bis 200°C,
- Halten der Temperatur von 200°C für 30 Minuten (dritte Temperaturrampe),
- weiteres Aufheizen bis 280°C,
- Halten der Temperatur von 280°C für 30 Minuten (vierte Temperaturrampe),
- weiteres Aufheizen bis 300°C,
- Halten der Temperatur von 300°C für 30 Minuten (fünfte Temperaturrampe),
- weiteres Aufheizen bis 340°C und Fortsetzen der thermischen Behandlung für weitere 150 Minuten,
- Abkühlen lassen des Ofens auf Raumtemperatur.

Während der Abkühlphase wurde der H₂-Durchfluss gestoppt und es wurde stattdessen Stickstoff in den Ofen eingeleitet.

Ein Teil des erhaltenen Palladium(0)-Pulvers wurde einer thermogravimetrischen Analyse unterzogen (TG-Gerät: Netzsch TG 209). Die Aufheizrate betrug 10°C/min. und die Probe wurde in einer Luftatmosphäre bis zu einer Temperatur von 990°C erhitzt. Die Probe zeigte eine Massenzunahme von 14,2 Gew%. Dem Palladium(0)-Pulver wurde noch eine zweite Probe entnommen und erneut unter identischen Bedingungen einer thermogravimetrischen Analyse unterzogen. Die Probe zeigte eine Massenzunahme von 14,1 Gew%.

Das verbleibende Palladium(0)-Pulver wurde hinsichtlich seiner Aktivität bei der Herstellung von Palladiumacetat getestet. Hierzu wurde analog Vergleichsbeispiel 1 vorgegangen, d.h.:
30 g des Palladium(0)-Ausgangspulvers wurden mit 30 ml Essigsäureanhydrid und 300 ml Essigsäure versetzt. Danach erfolgte die Zugabe von Salpetersäure.

Auch ohne externes Erhitzen zeigt sich eine NOₓ-Entwicklung und das Palladium(0)-Pulver reagiert mit Essigsäure und Salpetersäure zu Palladiumacetat. Dies belegt, dass das erfindungsgemäße Palladium(0)-Pulver eine sehr hohe Aktivität aufweist.

### Vergleichsbeispiel 2

Das Palladium(0)-Ausgangspulver wurde in einen Rohrofen gegeben. Man ließ Wasserstoff in den Ofen einströmen. Der H₂-Durchfluss betrug 2 m³/h. Nach der Ausbildung der Wasserstoffgasatmosphäre wurde der Ofen mit dem folgenden Temperaturprogramm auf eine Maximaltemperatur von 380°C aufgeheizt:
- Aufheizen bis 100°C,
- Halten der Temperatur von 100°C für 60 Minuten (erste Temperaturrampe),
- weiteres Aufheizen bis 150°C,
- Halten der Temperatur von 150°C für 30 Minuten (zweite Temperaturrampe),
- weiteres Aufheizen bis 200°C,
- Halten der Temperatur von 200°C für 30 Minuten (dritte Temperaturrampe),
- weiteres Aufheizen bis 280°C,
- Halten der Temperatur von 280°C für 30 Minuten (vierte Temperaturrampe),
- weiteres Aufheizen bis 300°C,
- Halten der Temperatur von 300°C für 30 Minuten (fünfte Temperaturrampe),
- weiteres Aufheizen bis 380°C und Fortsetzen der thermischen Behandlung für weitere 150 Minuten,
- Abkühlen lassen des Ofens auf Raumtemperatur.

Während der Abkühlphase wurde der H₂-Durchfluss gestoppt und es wurde stattdessen Stickstoff in den Ofen eingeleitet.

Ein Teil des erhaltenen Palladium(0)-Pulvers wurde einer thermogravimetrischen Analyse unterzogen (TG-Gerät: Netzsch TG 209). Die Aufheizrate betrug 10°C/min und die Probe wurde in einer Luftatmosphäre bis zu einer Temperatur von 990°C erhitzt. Die Probe zeigte eine Massenzunahme von 11,9 Gew%.

Das verbleibende Palladium(0)-Pulver wurde hinsichtlich seiner Aktivität bei der Herstellung von Palladiumacetat getestet. Hierzu wurde analog Vergleichsbeispiel 1 und Beispiel 1 vorgegangen, d.h.:
30 g des Palladium(0)-Ausgangspulvers wurden mit 30 ml Essigsäureanhydrid und 300 ml Essigsäure versetzt. Danach erfolgte die Zugabe von Salpetersäure.

Nur durch zusätzliches externes Erhitzen auf etwa 80°C zeigt sich eine NOx-Entwicklung und das Palladium(0)-Pulver reagiert mit Essigsäure und Salpetersäure zu Palladiumacetat.

## Patentansprüche

1. Verfahren zur Herstellung von Palladium(0)-Pulver, wobei ein Palladium(0)-Ausgangspulver in einer Wasserstoffgasatmosphäre in einem Ofen einer thermischen Behandlung bei einer Temperatur im Bereich von 230°C bis 370°C unterzogen wird.

2. Verfahren nach Anspruch 1, wobei das Palladium(0)-Ausgangspulver durch Reduktion einer Pd(II)-Verbindung oder einer Pd(IV)-Verbindung erhalten wird.

3. Verfahren nach Anspruch 1 oder 2, wobei der Wasserstoffgehalt der Wasserstoffgasatmosphäre mindestens 5 Vol%, bezogen auf die Gesamtmenge der in der Wasserstoffgasatmosphäre vorliegenden Gase, beträgt.

4. Verfahren nach einem der vorstehenden Ansprüche, wobei die Wasserstoffgasatmosphäre durch ein kontinuierliches Zuführen von Wasserstoff in den Ofen erzeugt wird.

5. Verfahren nach einem der vorstehenden Ansprüche, wobei das Aufheizen des Ofens durch eine oder mehrere Temperaturrampen unterbrochen wird.

6. Verfahren nach einem der vorstehenden Ansprüche, wobei die thermische Behandlung des Palladium(0)-Ausgangspulvers bei einer Temperatur im Bereich von 230°C bis 360°C oder von 280°C bis 350°C erfolgt.

7. Palladium(0)-Pulver, das beim Aufheizen an Luft bis zu einer Temperatur von 990°C eine Massenzunahme von mindestens 13,0 Gew% aufweist.

8. Verwendung des Palladium(0)-Pulvers nach Anspruch 7 als Edukt für die Herstellung eines Palladiumsalzes.

9. Verfahren zur Herstellung eines Palladiumsalzes, umfassend
(i) die Bereitstellung eines Palladium(0)-Pulvers durch das Verfahren nach einem der Ansprüche 1-6,
(ii) die Umsetzung des Palladium(0)-Pulvers mit einer Mineralsäure.

10. Verfahren nach Anspruch 9, wobei die Mineralsäure Salpetersäure, Schwefelsäure, Salzsäure oder ein Gemisch aus mindestens zwei dieser Mineralsäuren ist.

11. Das Verfahren nach Anspruch 9 oder 10, wobei die Umsetzung in Schritt (ii) in Anwesenheit einer Carbonsäure oder eines Carbonsäureanhydrids oder eines Gemisches davon erfolgt.

## Claims

1. Process for preparing palladium(0) powder, wherein a palladium(0) starting powder is subjected to a thermal treatment at a temperature in the range from 230°C to 370°C in a hydrogen gas atmosphere in an oven.

2. Process according to Claim 1, wherein the palladium(0) starting powder is obtained by reduction of a Pd(II) compound or of a Pd(IV) compound.

3. Process according to Claim 1 or 2, wherein the hydrogen content of the hydrogen gas atmosphere is at least 5 vol%, based on the total amount of the gases present in the hydrogen gas atmosphere.

4. Process according to any of the preceding claims, wherein the hydrogen gas atmosphere is generated by continuous feeding of hydrogen into the oven.

5. Process according to any of the preceding claims, wherein the heating of the oven is interrupted by one or more temperature ramps.

6. Process according to any of the preceding claims, wherein the thermal treatment of the palladium(0) starting powder takes place at a temperature in the range from 230°C to 360°C or from 280°C to 350°C.

7. Palladium(0) powder which on heating in air up to a temperature of 990°C exhibits a mass increase of at least 13.0 wt%.

8. Use of the palladium(0) powder according to Claim 7 as starting material for the preparation of a palladium salt.

9. Process for preparing a palladium salt, comprising
(i) providing a palladium(0) powder by the process according to any of Claims 1-6,
(ii) reacting the palladium(0) powder with a mineral acid.

10. Process according to Claim 9, wherein the mineral acid is nitric acid, sulfuric acid, hydrochloric acid or a mixture of at least two of these mineral acids.

11. Process according to Claim 9 or 10, wherein the reacting in step (ii) takes place in the presence of a carboxylic acid or a carboxylic anhydride or a mixture thereof.

## Revendications

1. Procédé pour la préparation de poudre de palladium (0), dans lequel une poudre de départ de palladium (0) est soumise, dans une atmosphère d'hydrogène gazeux dans un four, à un traitement thermique à une température dans la plage de 230°C à 370°C.

2. Procédé selon la revendication 1, dans lequel la poudre de départ de palladium (0) est obtenue par réduction d'un composé du Pd (II) ou d'un composé du Pd (IV) .

3. Procédé selon la revendication 1 ou 2, dans lequel la teneur en hydrogène de l'atmosphère d'hydrogène gazeux représente au moins 5% en volume, par rapport à la quantité totale des gaz se trouvant dans l'atmosphère d'hydrogène gazeux.

4. Procédé selon l'une quelconque des revendications précédentes, dans lequel l'atmosphère d'hydrogène gazeux est générée par une introduction continue d'hydrogène dans le four.

5. Procédé selon l'une quelconque des revendications précédentes, dans lequel le chauffage du four est interrompu par une ou plusieurs rampes de température.

6. Procédé selon l'une quelconque des revendications précédentes, dans lequel le traitement thermique de la poudre de départ de palladium (0) a lieu à une température dans la plage de 230°C à 360°C ou de 280°C à 350°C.

7. Poudre de palladium (0) qui présente une augmentation de masse d'au moins 13,0% en poids lors du chauffage à l'air jusqu'à une température de 990°C.

8. Utilisation de la poudre de palladium (0) selon la revendication 7 comme produit de départ pour la préparation d'un sel de palladium.

9. Procédé pour la préparation d'un sel de palladium, comprenant
(i) la préparation d'une poudre de palladium (0) par le procédé selon l'une quelconque des revendications 1-6,
(ii) la transformation de la poudre de palladium (0) avec un acide minéral.

10. Procédé selon la revendication 9, dans lequel l'acide minéral est l'acide nitrique, l'acide sulfurique, l'acide chlorhydrique ou un mélange d'au moins deux de ces acides minéraux.

11. Procédé selon la revendication 9 ou 10, dans lequel la transformation dans l'étape (ii) a lieu en présence d'un acide carboxylique ou d'un anhydride d'acide carboxylique ou d'un mélange de ceux-ci.
